(19) Europäisches Patentamt
European Patent Office
Office européen des brevets

(11) Veröffentlichungsnummer : **0 167 817**
**B1**

(12) # EUROPÄISCHE PATENTSCHRIFT

(45) Veröffentlichungstag der Patentschrift : **20.09.89**

(51) Int. Cl.[4] : **C 07 D487/04**, A 61 K 31/505 // (C07D487/04, 239:00, 239:00)

(21) Anmeldenummer : **85106900.5**

(22) Anmeldetag : **04.06.85**

(54) **Neue 8-Alkylthio-2-piperazino-pyrimido[5,4-d]pyrimidine, ihre Herstellung und diese Verbindungen enthaltende Arzneimittel.**

(30) Priorität : **22.06.84 DE 3423092**

(43) Veröffentlichungstag der Anmeldung : **15.01.86 Patentblatt 86/03**

(45) Bekanntmachung des Hinweises auf die Patenterteilung : **20.09.89 Patentblatt 89/38**

(84) Benannte Vertragsstaaten : **AT BE CH DE FR GB IT LI LU NL SE**

(56) Entgegenhaltungen :
**EP--A-- 0 023 559**
**DE--A-- 1 116 676**
**Die Akte enthält technische Angaben, die nach dem Eingang der Anmeldung eingereicht wurden und die nicht in dieser Patentschrift enthalten sind.**

(73) Patentinhaber : **Dr. Karl Thomae GmbH**
**Postfach 1755**
**D-7950 Biberach (Riss) (DE)**

(72) Erfinder : **Roch, Josef, Dr. Dipl.-Chem.**
**Stecherweg 19**
**D-7950 Biberach 1 (DE)**
Erfinder : **Heckel, Armin, Dr. Dipl.-Chem.**
**Lamparterweg 1**
**D-7950 Biberach 1 (DE)**
Erfinder : **Nickl, Josef, Dr. Dipl.-Chem.**
**Silcherstrasse 8**
**D-7950 Biberach 1 (DE)**
Erfinder : **Müller, Erich, Dr. Dipl.-Chem.**
**Talfeldstrasse 34**
**D-7950 Biberach 1 (DE)**
Erfinder : **Narr, Berthold, Dr. Dipl.-Chem.**
**Ober Au 5**
**D-7950 Biberach 1 (DE)**
Erfinder : **Zimmermann, Rainer, Dr. Dipl.-Biochem.**
**Laurenbühlstrasse 17**
**D-7951 Mittelbiberach (DE)**
Erfinder : **Weisenberger, Johannes, Dr. Dipl.-Chem.**
**Haydnweg 5**
**D-7950 Biberach 1 (DE)**

**EP 0 167 817 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

## Beschreibung

In der EP-A-0 023 559 wurden bereits dreifach substituierte 2-Piperazino-pyrimido[5,4-d]pyrimidine beschrieben, welche wertvolle pharmakologische Eigenschaften aufweisen, insbesondere eine antithrombotische Wirkung, eine PDE-Hemmwirkung und eine Hemmwirkung auf die Aggregation von in die Blutbahn geschwemmten Krebszellen.

Überraschenderweise wurde nun gefunden, daß die neuen 8-Alkylthio-2-piperazino-pyrimido[5,4-d]pyrimidine der allgemeinen Formel I

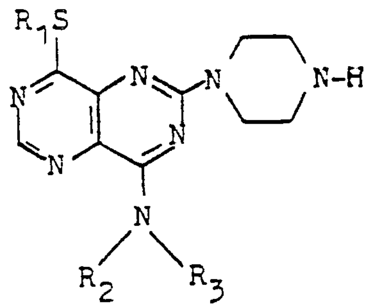

(I)

welche sich immer durch den Substituenten in 4-Stellung von den Verbindungen der EP-A-0 023 559 unterscheiden, überlegene pharmakologische Eigenschaften aufweisen, insbesondere aufgrund ihrer selektiven Tumor-PDE-hemmenden Wirkung, eine metastasenhemmende Wirkung und eine Hemmwirkung auf das Tumorwachstum.

Gegenstand der Erfindung sind die neun Verbindungen der obigen allgemeinen Formel I, deren Säureadditionssalze, insbesondere deren Säureadditionssalze mit physiologisch verträglichen anorganischen oder organischen Säuren, diese Verbindungen bzw. deren physiologisch verträgliche Säureadditionssalze enthaltende Arzneimittel und Verfahren zu ihrer Herstellung.

In der obigen allgemeinen Formel I bedeutet:

$R_1$ eine Alkylgruppe mit 1 bis 3 Kohlenstoffatomen,

$R_2$ ein Wasserstoffatom, eine ab dem Kohlenstoff 2 gegebenenfalls durch eine Hydroxygruppe substituierte Alkylgruppe mit 1 bis 6 Kohlenstoffatomen, eine Cycloalkylgruppe mit 5 bis 7 Kohlenstoffatomen, eine Allyl-, Phenyl- oder Benzylgruppe,

$R_3$ eine Allylgruppe, eine ab dem Kohlenstoffatom 2 gegebenenfalls durch eine Hydroxygruppe substituierte Alkylgruppe mit 1 bis 6 Kohlenstoffatomen oder eine Cycloalkylgruppe mit 5 bis 7 Kohlenstoffatomen oder

$R_2$ und $R_3$ zusammen mit dem dazwischenliegenden Stickstoffatom eine geradkettige Alkyleniminogruppe mit 2 bis 8 Kohlenstoffatomen.

Für die bei der Definition der Reste $R_1$ bis $R_3$ eingangs erwähnten Bedeutungen kommt beispielsweise für $R_1$ die Bedeutung der Methyl-, Ethyl-, n-Propyl- oder Isopropylgruppe und

für

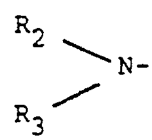

die der Methylamino-, Ethylamino-, n-Propylamino-, Isopropylamino-, n-Butylamino-, Isobutylamino-, tert.Butylamino-, n-Pentylamino-, Isopentylamino-, tert.Pentylamino-, n-Hexylamino-, Dimethylamino-, Diethylamino-, Di-n-propylamino-, Di-n-butylamino-, Di-n-pentylamino-, Di-n-hexylamino-, Diisopropylamino-, N-Methyl-ethylamino-, N-Methyl-isopropylamino-, N-Ethyl-n-propylamino-, Cyclopentylamino-, Cyclohexylamino-, Cycloheptylamino-, Dicyclopentylamino-, Dicyclohexylamino-, Dicycloheptylamino-, N-Cyclopentyl-cyclohexylamino-, 2-Hydroxyethylamino-, 3-Hydroxy-n-propylamino-, 4-Hydroxy-n-butylamino-, 6-Hydroxy-n-hexylamino-, 2-Hydroxy-n-propylamino-, Di(2-Hydroxyethyl)-amino-, Di(3-Hydroxy-n-propyl)-amino-, Di(2-Hydroxy-n-propyl)-amino-, Di(6-Hydroxy-n-hexyl)-amino-, N-Methyl-cyclopentylamino-, N-Methyl-cyclohexylamino-, N-Ethyl-cyclohexylamino-, N-Isopropyl-cycloheptylamino-, N-n-Hexyl-cyclohexylamino-, N-(2-Hydroxyethyl)-cyclohexylamino-, N-(2-Hydroxy-n-propyl)-cyclopentylamino-, N-(3-Hydroxy-n-propyl)-cycloheptylamino-, N-(6-Hydroxy-n-hexyl)-cyclohexylamino-, Allylamino-, Phenylamino-, Benzylamino-, Diallylamino-, N-Methyl-allylamino-, N-Ethyl-allylamino-, N-Isopropyl-allylamino-, N-(2-Hydroxyethyl)-allylamino-, N-Methyl-phenylamino-, N-Ethyl-phenylamino-, N-n-Hexyl-phenylamino-, N-(2-Hydroxy-ethyl)-phenylamino-, N-(3-Hydroxy-n-propyl)-phenylamino-, N-(6-Hydroxy-n-hexyl)-phenylamino-, Dibenzylamino-, N-Methylbenzylamino-, N-Ethylbenzylamino-, N-n-Propyl-benzylamino-, N-n-Hexyl-benzylamino-, N-(2-Hydroxyethyl)-benzylamino-, N-(3-Hydroxy-n-propyl)-benzylamino-, N-(6-Hydroxy-n-hexyl)-benzylamino-, N-Allyl-cyclohexylamino-, N-Allyl-benzylamino-, Dimethylenimino-, Trimethylenimino-, Pyrrolidino-, Piperidino-, Hexamethylenimino-, Heptamethylenimino- oder Octamethyleniminogruppe in Betracht.

Bevorzugte Verbindungen der obigen allgemeinen Formel I sind jedoch diejenigen, in denen

$R_1$ wie eingangs definiert ist,

$R_2$ ein Wasserstoffatom, eine Allylgruppe oder eine Alkylgruppe mit 1 bis 4 Kohlenstoffatomen,

$R_3$ eine Alkylgruppe mit 1 bis 6 Kohlenstoffatomen, eine Allyl-, Cyclohexyl-, Phenyl-, Benzyl-, 2-Hydroxyethyl-, 2-Hydroxy-n-propyl- oder 3-Hydroxy-n-propylgruppe oder

$R_2$ und $R_3$ zusammen mit dem dazwischenliegenden Stickstoffatom eine Pyrrolidino-, Piperidino-, Hexamethylenimino- oder Heptamethyleniminogruppe bedeuten, insbesondere jedoch diejenigen Verbindungen der allgemeinen Formel I, in denen

$R_1$ eine Methylgruppe

$R_2$ ein Wasserstoffatom, eine Methyl- oder Ethylgruppe,

$R_3$ eine Alkylgruppe mit 1 bis 6 Kohlenstoffatomen oder

$R_2$ und $R_3$ zusammen mit dem dazwischenliegenden Stickstoffatom eine Pyrrolidino-, Piperidino- oder Hexamethyleniminogruppe bedeuten, und deren Säureadditionssalze, insbesondere deren physiologisch verträgliche Säureadditionssalze.

Erfindungsgemäß erhält man die neuen Verbindungen der obigen allgemeinen Formel I durch Umsetzung eines Pyrimido[5,4-d]pyrimidins der allgemeinen Formel II

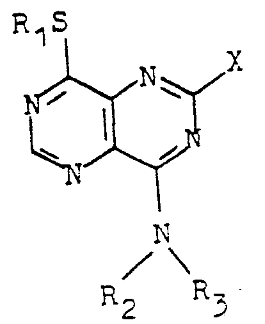

(II)

in der

$R_1$ bis $R_3$ wie eingangs definiert sind und

X eine nukleofuge Austrittsgruppe wie ein Halogenatom, z. B. ein Chlor- oder Bromatom, eine substituierte Hydroxygruppe, z. B. die Phenoxygruppe, oder eine Sulfonylgruppe, z. B. die Methylsulfonylgruppe, darstellt, mit einem Piperazin der allgemeinen Formel III

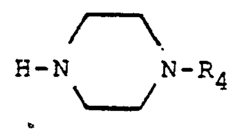

(III)

in der

$R_4$ ein Wasserstoffatom oder eine leicht abspaltbare Schutzgruppe wie die Trimethylsilylgruppe, eine Kohlensäureestergruppe, z. B. die Carbäthoxygruppe, oder eine Alkanoylgruppe, z. B. die Formyl- oder Acetylgruppe, darstellt und erforderlichenfalls anschließende Abspaltung eines verwendeten Schutzrestes.

Die Umsetzung wird zweckmäßigerweise in einem inerten Lösungsmittel wie Aceton, Methylenchlorid, Chloroform, Tetrahydrofuran, Dioxan, Dimethylformamid oder Dimethylsulfoxid gegebenenfalls in Gegenwart einer anorganischen Base wie Natriumcarbonat oder Kaliumhydroxid oder einer tertiären organischen Base wie Triethylamin oder Pyridin, wobei letzere gleichzeitig auch als Lösungsmittel verwendet werden können, bei Temperaturen zwischen 0 und 120 °C, vorzugsweise jedoch bei Temperaturen zwischen 20 und 50 °C, durchgeführt.

Die Umsetzung kann jedoch auch ohne Lösungsmittel oder in einem Überschuß der eingesetzten Verbindungen der allgemeinen Formel III durchgeführt werden.

Die gegebenenfalls anschließende Abspaltung eines verwendeten Schutzrestes erfolgt zweckmäßigerweise hydrolytisch in Gegenwart einer Säure oder Base in einem wäßrigen Lösungsmittel wie Wasser/Methanol oder Wasser/Ethanol und vorzugsweise bei der Siedetemperatur des Reaktionsgemisches.

Die so erhaltenen Verbindungen der allgemeinen Formel I lassen sich gewünschtenfalls anschließend in ihre Säureadditionssalze, insbesondere in ihre Säureadditionssalze mit physiologisch verträglichen anorganischen oder organischen Säuren überführen. Als Säuren kommen beispielsweise Salzsäure, Bromwasserstoffsäure, Schwefelsäure, Phosphorsäure, Methansulfonsäure, p-Toluolsulfonsäure, Essigsäure, Milchsäure, Citronensäure, Weinsäure, Bernsteinsäure, Maleinsäure, Fumarsäure oder Salicylsäure in Betracht.

Die als Ausgangsstoffe verwendeten Verbindungen der allgemeinen Formel II erhält man beispielsweise durch stufenweisen Ersatz der Chloratome des 2,4,8-Trichlor-pyrimido[5,4-d]pyrimidins, wobei zuerst das Chloratom in Stellung 4, dann das in Stellung 8 nach an sich bekannten Verfahren ausgetauscht wird.

Wie bereits eingangs erwähnt, weisen die neuen Verbindungen der allgemeinen Formel I und deren physiologisch verträgliche Säureadditionssalze wertvolle pharmakologische Eigenschaften auf, neben einer antithrombotischen Wirkung insbesondere jedoch aufgrund ihrer selektiven Tumor-PDE-hemmenden Wirkung eine metastasenhemmende und tumorwachstumshemmende Wirkung.

Beispielsweise wurden die Verbindungen
A = 8-Methylthio-2-piperazino-4-pyrrolidino-pyrimido[5,4-d]pyrimidin,
B = 4-Diethylamino-8-methylthio-2-piperazino-pyrimido[5,4-d]pyrimidin,
C = 4-n-Hexylamino-8-methylthio-2-piperazino-pyrimido[5,4-d]pyrimidin und
D = 4-Hexamethylenimino-8-methylthio-2-piperazino-pyrimido[5,4-d]pyrimidin
auf ihre Hemmwirkung auf Tumorzellen wie folgt untersucht :

a) Enzymgewinnung :

Die Phosphodiesterase wurde aus B16-Melanomgewebe von Mäusen durch Zentrifugation des Gewebehomogenates bei 5.000 × g (15 Minuten, 4 °C) gewonnen. Die Homogenisation der Gewebe erfolgte durch wiederholtes Frieren/Auftauen und Homogenisation nach Potter-Elvehjem bzw. durch Ultraschall. Der die PDE enthaltende Homogenat-Überstand wurde portioniert bei — 25 °C tiefgefroren gelagert.

Die Gewinnung der Phosphodiesterase aus Humanthrombozyten erfolgte analog.

b) Bestimmung der PDE-Hemmung (PDE-assay) :

Die Bestimmung der PDE-Hemmung durch die Prüfsubstanzen erfolgte mit 1 µmol/l $^3$H-cAMP als Substrat. Die PDE-Hemmung wurde durch Messung des Abbaus des eingesetzten Substrats $^3$H-cAMP zu $^3$H-AMP im Vergleich zur Kontrolle ohne Prüfsubstanz erfaßt. Das gebildete $^3$H-AMP wurde durch eine Zinksulfat-Bariumhydroxid-Fällung vom verbliebenen $^3$H-cAMP abgetrennt.

Die Berechnung der $ED_{50}$ als die Konzentration, die die PDE-Aktivität um 50 % hemmte, erfolgte mittels linearer Regressionsanalyse.

| Substanz | PDE-Hemmung $ED_{50}$ (µMol/l) Human-Thrombozyten | $B_{16}$-Tumorzelle |
|---|---|---|
| A | 1,6 | 0,066 |
| B | 1,3 | 0,037 |
| C | 1,2 | 0,12 |
| D | 1,7 | 0,028 |

Außerdem weisen die neuen Verbindungen eine gute Verträglichkeit auf. So verendete beispielsweise bei der Applikation der Substanz A an der Maus bei einer Dosis von 20 mg/kg i.v. bzw. 100 mg/kg p.o. keines der jeweils eingesetzten 10 Tiere.

Aufgrund ihrer pharmakologischen Eigenschaften eignen sich die Verbindungen der allgemeinen Formel I sowie deren Säureadditionssalze mit physiologisch verträglichen anorganischen oder organischen Säuren zur Prophylaxe thrombo-embolischer Erkrankungen wie Coronarinfarkt, Cerebralinfarkt, sogn. transient ischaemic attacks, Amaurosis fugax, zur Prophylaxe der Arteriosklerose, zur Metastasenprophylaxe und zur Hemmung des Tumorwachstums.

Die zur Erzielung einer entsprechenden Wirkung erforderlichen Dosierung beträgt zweckmäßigerweise 2- bis 4-mal täglich 0,1 bis 4 mg/kg Körpergewicht, vorzugsweise 0,2 bis 3 mg/kg Körpergewicht. Hierzu lassen sich die erfindungsgemäß hergestellten Verbindungen der allgemeinen Formel I sowie ihre physiologisch verträglichen Säureadditionssalze mit anorganischen oder organischen Säuren, gegebenenfalls in Kombination mit anderen Wirksubstanzen, zusammen mit einem oder mehreren inerten üblichen Trägerstoffen und/oder Verdünnungsmitteln, z. B. mit Maisstärke, Milchzucker, Rohrzucker, mikrokristalliner Cellulose, Magnesiumstearat, Polyvinylpyrrolidon, Citronensäure, Weinsäure, Wasser, Wasser/Äthanol, Wasser/Glycerin, Wasser/Sorbit, nichtionische Tenside wie z. B. Polyoxyäthylen-Fettsäureester, Wasser-Polyäthylenglykol, Propylenglykol, Cetylstearylalkohohl, Carboxymethylcellulose oder fetthaltige Substanzen wie Hartfett oder deren geeignete Gemische, in übliche galenische Zubereitungen

wie Tabletten, Dragées, Kapseln, Pulver, Suspensionen, Tropfen, Ampullen, Säfte oder Zäpfchen einarbeiten.

Die nachfolgenden Beispiele sollen die Erfindung näher erläutern :

Beispiel A

2,8-Dichlor-4-pyrrolidino-pyrimido[5,4-d]pyrimidin

4,7 g (0,02 Mol) 2,4,8-Trichlor-pyrimido[5,4-d]pyrimidin werden in 200 ml Chloroform gelöst, auf 5 °C gekühlt und mit 3,4 g (0,04 Mol) Natriumhydrogencarbonat in 40 ml Wasser versetzt. Anschließend tropft man 1,65 ml (0,02 Mol) Pyrrolidin in 20 ml Chloroform zu und rührt die Lösung 15 Minuten bei 5 °C und 30 Minuten bei Raumtemperatur. Dann wird die organische Phase abgetrennt, mit 100 ml Wasser gewaschen, über Natriumsulfat getrocknet und einrotiert.

Ausbeute : 4,7 g (87 % der Theorie),
Schmelzpunkt : 144-146 °C (Ethanol)
Analog erhält man folgende Verbindungen :

2,8-Dichlor-4-piperidino-pyrimido[5,4-d]pyrimidin
Schmelzpunkt : 114-117 °C (Ethanol)
2,8-Dichlor-4-hexamethylenimino-pyrimido[5,4-d]pyrimidin
Schmelzpunkt : 121-123 °C (Methanol)
2,8-Dichlor-4-dimethylamino-pyrimido[5,4-d]pyrimidin
Schmelzpunkt : 161-163 °C (Ethylacetat)
2,8-Dichlor-4-diethylamino-pyrimido[5,4-d]pyrimidin
Schmelzpunkt : 113-114 °C (Ethanol)
2,8-Dichlor-4-di-n-propylamino-pyrimido[5,4-d]pyrimidin
Schmelzpunkt : 51-52 °C
2,8-Dichlor-4-di-n-butylamino-pyrimido[5,4-d]pyrimidin
Schmelzpunkt : 47-49 °C
2,8-Dichlor-4-(N-cyclohexyl-methylamino)-pyrimido[5,4-d]pyrimidin
Schmelzpunkt : 143-145 °C
4-(N-Benzyl-methylamino)-2,8-dichlor-pyrimido[5,4-d]pyrimidin
Schmelzpunkt : 136-138 °C
2,8-Dichlor-4-(N-methyl-phenylamino)-pyrimido[5,4-d]pyrimidin
Schmelzpunkt : 208-209 °C
2,8-Dichlor-4-(N-ethyl-3′-hydroxy-n-propylamino)-pyrimido[5,4-d]pyrimidin
Schmelzpunkt : 70-72 °C
2,8-Dichlor-4-methylamino-pyrimido[5,4-d]pyrimidin
Schmelzpunkt : 196-197 °C (Ethylacetat/Methanol)
2,8-Dichlor-4-n-hexylamino-pyrimido[5,4-d]pyrimidin
Schmelzpunkt : 78-80 °C (Petrolether)
2,8-Dichlor-4-heptamethylenimino-pyrimido[5,4-d]pyrimidin
Schmelzpunkt : 129-131 °C
2,8-Dichlor-4-(N-methyl-ethylamino)-pyrimido[5,4-d]pyrimidin
Schmelzpunkt : 108-110 °C
4-Diallylamino-2,8-dichlor-pyrimido[5,4-d]pyrimidin
Schmelzpunkt : 127-129 °C

Beispiel B

2-Chlor-8-methylthio-4-pyrrolidino-pyrimido[5,4-d]pyrimidin

4,7 g (0,02 Mol) 2,8-Dichlor-4-pyrrolidino-pyrimido[5,4-d]pyrimidin werden in 200 ml Dioxan/Methanol (1 : 1) gelöst und mit einer Lösung von 0,46 g (0,02 Mol) Natrium und 2,2 ml (0,04 Mol) Methylmercaptan in 50 ml Methanol bei 5 °C versetzt. Man rührt 15 Minuten bei 5 °C und anschließend 30 Minuten bei Raumtemperatur. Die erhaltene Suspension wird in 500 ml Wasser gegeben. Der ausfallende Niederschlag wird abgesaugt, mit Wasser gewaschen und getrocknet.

Ausbeute : 96,2 % der Theorie,
Schmelzpunkt : 188-191 °C (Essigester)

Beispiel C

2-Chlor-8-methylthio-4-piperidino-pyrimido[5,4-d]pyrimidin

4,26 g (15 mMol) 2,8-Dichlor-4-piperidino-pyrimido[5,4-d]pyrimidin werden in 150 ml Aceton gelöst

und auf 0 °C abgekühlt. Bei dieser Temperatur wird eine Lösung von 0,35 g (15 mMol) Natrium in 25 ml Methanol mit 0,85 ml (15 mMol) Methylmercaptan zugetropft. Anschließend wird 30 Minuten unter Kühlung gerührt. Nach Eindampfen der erhaltenen Lösung wird der Rückstand in 300 ml Methylenchlorid aufgenommen. Man wäscht mit 2 × 100 ml Wasser, trocknet die organische Phase über Natriumsulfat und engt ein.

Ausbeute : 3,9 g (88 % der Theorie),
Schmelzpunkt : 135-136 °C (Ethanol)
Analog erhält man folgende Verbindungen :

2-Chlor-8-ethylthio-4-piperidino-pyrimido[5,4-d]pyrimidin
Schmelzpunkt : 110-111 °C (Ethanol)
2-Chlor-4-piperidino-8-n-propylthio-pyrimido[5,4-d]pyrimidin
Schmelzpunkt : 107-109 °C (Ethanol)
2-Chlor-4-hexamethylenimino-8-methylthio-pyrimido[5,4-d]pyrimidin
Schmelzpunkt : 173-175 °C (Dioxan)
2-Chlor-4-dimethylamino-8-methylthio-pyrimido[5,4-d]pyrimidin
Schmelzpunkt : 166-168 °C (Ethylacetat)
2-Chlor-4-diethylamino-8-methylthio-pyrimido[5,4-d]pyrimidin
Schmelzpunkt : 90-91 °C (Methanol)
2-Chlor-4-di-n-propylamino-8-methylthio-pyrimido[5,4-d]pyrimidin
Schmelzpunkt : 92-94 °C
2-Chlor-4-di-n-butylamino-8-methylthio-pyrimido[5,4-d]pyrimidin
Schmelzpunkt : 42-44 °C
2-Chlor-4-(N-cyclohexyl-methylamino)-8-methylthio-pyrimido[5,4-d]pyrimidin
Schmelzpunkt : 135-137 °C (Ethanol)
4-(N-Benzyl-methylamino)-2-chlor-8-methylthio-pyrimido[5,4-d]pyrimidin
Schmelzpunkt : 93 °C (Sintern)
2-Chlor-4-(N-methyl-phenylamino)-8-methylthio-pyrimido[5,4-d]pyrimidin
Schmelzpunkt : 183-184 °C (Methanol)
2-Chlor-4-(N-ethyl-3′-hydroxy-n-propylamino)-8-methylthio-pyrimido[5,4-d]pyrimidin
Sintert ab 66 °C
2-Chlor-4-methylamino-8-methylthio-pyrimido[5,4-d]pyrimidin
Schmelzpunkt : 233-234 °C (Dioxan/Wasser 1 : 1)
2-Chlor-4-hexylamino-8-methylthio-pyrimido[5,4-d]pyrimidin
Schmelzpunkt : 80-82 °C (Methanol)
2-Chlor-4-heptamethylenimino-8-methylthio-pyrimido[5,4-d]pyrimidin
Schmelzpunkt : 124-125 °C (Methanol)
2-Chlor-4-(N-methyl-ethylamino)-8-methylthio-pyrimido[5,4-d]pyrimidin
Schmelzpunkt : 107-109 °C
2-Chlor-4-diallylamino-8-methylthio-pyrimido[5,4-d]pyrimidin
Schmelzpunkt : 83-84 °C (Methanol)

## Beispiel 1

8-Methylthio-2-piperazino-4-pyrrolidino-pyrimido[5,4-d]pyrimidin

2,8 g (0,01 Mol) 2-Chlor-8-methylthio-4-pyrrolidino-pyrimido[5,4-d]pyrimidin werden in 300 ml Dimethylsulfoxid warm gelöst und mit einer Lösung von 4,3 g (0,05 Mol) Piperazin versetzt. Nach 1-stündigem Rühren bei Raumtemperatur wird die Lösung in 1,5 l Wasser gegossen. Der ausfallende Niederschlag wird abgesaugt, mit Wasser gewaschen und getrocknet.

Ausbeute : 3 g (90 % der Theorie).

Zur weiteren Reinigung wird in 500 ml 0,1 N Salzsäure gelöst und mit konz. Ammoniak wieder ausgefällt.

Schmelzpunkt : 188-189 °C.

## Beispiel 2

8-Methylthio-4-piperidino-2-piperazino-pyrimido[5,4-d]pyrimidin

2,0 g (6,8 mMol) 2-Chlor-8-methylthio-4-piperidino-pyrimido[5,4-d]pyrimidin werden in 70 ml Dimethylsulfoxid warm gelöst und mit einer Lösung von 6 g Piperazin in 70 ml Dimethylsulfoxid 1 Stunde bei Raumtemperatur gerührt. Anschließend gießt man die Lösung in 1 l Wasser, saugt den abgeschiedenen Niederschlag ab und wäscht mit Wasser nach. Der Rückstand wird in 50 ml Methylenchlorid aufgenommen und mit 50 ml 0,05 N Natronlauge geschüttelt. Die organische Phase wird abgetrennt, über Natriumsulfat getrocknet und einrotiert.

Ausbeute : 1,1 g (47 % der Theorie),
Schmelzpunkt : 159-160 °C.

## Beispiel 3

8-Ethylthio-4-piperidino-2-piperazino-pyrimido[5,4-d]pyrimidin

Hergestellt analog Beispiel 2 aus 2-Chlor-8-ethylthio-4-piperidino-pyrimido[5,4-d]pyrimidin und Piperazin in Dimethylsulfoxid.
Ausbeute : 57 % der Theorie,
Schmelzpunkt : 130-131 °C.

## Beispiel 4

4-Piperidino-2-piperazino-8-n-propylthio-pyrimido[5,4-d]pyrimidin

Hergestellt analog Beispiel 2 aus 2-Chlor-4-piperidino-8-n-propylthio-pyrimido[5,4-d]pyrimidin und Piperazin in Dimethylsulfoxid.
Ausbeute : 69 % der Theorie,
Schmelzpunkt : 144-146 °C.

## Beispiel 5

4-Hexamethylenimino-8-methylthio-2-piperazino-pyrimido[5,4-d]pyrimidin

Hergestellt analog Beispiel 2 aus 2-Chlor-4-hexamethylenimino-8-methylthio-pyrimido[5,4-d]pyrimidin und Piperazin in Dimethylsulfoxid.
Ausbeute : 54 % der Theorie,
Schmelzpunkt : 124-126 °C (Methanol).

## Beispiel 6

4-Dimethylamino-8-methylthio-2-piperazino-pyrimido[5,4-d]pyrimidin

Hergestellt analog Beispiel 2 aus 2-Chlor-4-dimethylamino-8-methylthio-pyrimido[5,4-d]pyrimidin und Piperazin in Dimethylsulfoxid.
Ausbeute : 91 % der Theorie,
Schmelzpunkt : 166-168 °C (Ethylacetat).

## Beispiel 7

4-Diethylamino-8-methylthio-2-piperazino-pyrimido[5,4-d]pyrimidin

Hergestellt analog Beispiel 2 aus 2-Chlor-4-diethylamino-8-methylthio-pyrimido[5,4-d]pyrimidin und Piperazin in Dimethylsulfoxid.
Ausbeute : 69 % der Theorie,
Schmelzpunkt : 136 °C (Methanol ; Sintert ab 110 °C).

## Beispiel 8

4-Di-n-propylamino-8-methylthio-2-piperazino-pyrimido[5,4-d]pyrimidin

Hergestellt analog Beispiel 2 aus 2-Chlor-4-di-n-propylamino-8-methylthio-pyrimido[5,4-d]pyrimidin und Piperazin in Dimethylsulfoxid.
Ausbeute : 95 % der Theorie,
Schmelzpunkt : 78-80 °C.

## Beispiel 9

4-Di-n-butylamino-8-methylthio-2-piperazino-pyrimido[5,4-d]pyrimidin

Hergestellt analog Beispiel 2 aus 2-Chlor-4-di-n-butylamino-8-methylthio-pyrimido[5,4-d]pyrimidin und Piperazin in Dimethylsulfoxid.
Ausbeute : 42 % der Theorie,
Schmelzpunkt : 64-70 °C.

Beispiel 10

4-(N-Cyclohexyl-methylamino)-8-methylthio-2-piperazino-pyrimido[5,4-d]pyrimidin

Hergestellt analog Beispiel 2 aus 2-Chlor-4-(N-cyclohexylmethylamino)-8-methylthio-pyrimido[5,4-d]pyrimidin und Piperazin in Dimethylsulfoxid.
Ausbeute : 64 % der Theorie,
Schmelzpunkt : 153-154 °C (Ethylacetat).

Beispiel 11

4-(N-Benzyl-methylamino)-8-methylthio-2-piperazino-pyrimido[5,4-d]pyrimidin

Hergestellt analog Beispiel 2 aus 2-Chlor-4-(N-Benzyl-methylamino)-8-methylthio-pyrimido[5,4-d]pyrimidin und Piperazin in Dimethylsulfoxid.
Ausbeute : 45 % der Theorie,
Schmelzpunkt : 129-131 °C.

Beispiel 12

4-(N-Methyl-phenylamino)-8-methylthio-2-piperazino-pyrimido[5,4-d]pyrimidin

Hergestellt analog Beispiel 2 aus 2-Chlor-4-(N-methylphenylamino)-8-methylthio-pyrimido[5,4-d]pyrimidin und Piperazin in Dimethylsulfoxid.
Ausbeute : 68 % der Theorie,
Schmelzpunkt : 126-128 °C.

Beispiel 13

4-(N-Ethyl-3'-hydroxy-n-propylamino)-8-methylthio-2-piperazino-pyrimido[5,4-d]pyrimidin

Hergestellt analog Beispiel 2 aus 2-Chlor-4-(N-ethyl-3'-hydroxypropylamino)-8-methylthio-pyrimido[5,4-d]pyrimidin und Piperazin in Dimethylsulfoxid.
Ausbeute : 55 % der Theorie,
Schmelzpunkt : 123-125 °C.

Beispiel 14

4-Methylamino-8-methylthio-2-piperazino-pyrimido[5,4-d]pyrimidin

Hergestellt analog Beispiel 2 aus 2-Chlor-4-methylamino-8-methylthio-pyrimido[5,4-d]pyrimidin und Piperazin in Dimethylsulfoxid.
Ausbeute : 70 % der Theorie,
Schmelzpunkt : 193-194 °C (Wasser).

Beispiel 15

4-n-Hexylamino-8-methylthio-2-piperazino-pyrimido[5,4-d]pyrimidin

Hergestellt analog Beispiel 2 aus 2-Chlor-4-n-hexylamino-8-methylthio-pyrimido[5,4-d]pyrimidin und Piperazin in Dimethylsulfoxid.
Ausbeute : 95 % der Theorie,
Schmelzpunkt : 142-144 °C.

Beispiel 16

4-Heptamethylenimino-8-methylthio-2-piperazino-pyrimido[5,4-d]pyrimidin

Hergestellt analog Beispiel 2 aus 2-Chlor-4-heptamethylenimino-8-methylthio-pyrimido[5,4-d]pyrimidin und Piperazin in Dimethylsulfoxid.
Ausbeute : 82 % der Theorie,
Schmelzpunkt : 146-148 °C.

Beispiel 17

4-(N-Methyl-ethylamino)-8-methylthio-2-piperazino-pyrimido[5,4-d]pyrimidin

Hersgestellt analog Beispiel 2 aus 2-Chlor-4-(N-methyl-ethylamino)-8-methylthio-pyrimido[5,4-d]pyrimidin und Piperazin in Dimethylsulfoxid.
Ausbeute : 60 % der Theorie,
Schmelzpunkt : 207-209 °C.

Beispiel 18

4-Diallylamino-8-methylthio-2-piperazino-pyrimido[5,4-d]pyrimidin

Hergestellt analog Beispiel 2 aus 2-Chlor-4-diallylamino-8-methylthio-pyrimido[5,4-d]pyrimidin und Piperazin in Dimethylsulfoxid.
Ausbeute : 66 % der Theorie,
Schmelzpunkt des Hydrochlorids : 178-180 °C.
Analog den vorstehenden Beispielen werden folgende Verbindungen erhalten :

4-Dimethylenimino-8-methylthio-2-piperazino-pyrimido[5,4-d]pyrimidin
8-Methylthio-2-piperazino-4-trimethylenimino-pyrimido[5,4-d]pyrimidin
8-Methylthio-4-octamethylenimino-2-piperazino-pyrimido[5,4-d]pyrimidin

Beispiel I

Dragées mit 4 mg 8-Methylthio-2-piperazino-4-pyrrolidino-pyrimido[5,4-d]pyrimidin

Zusammensetzung:

1 Drageékern enthält:

| | | |
|---|---|---|
| Wirksubstanz | (1) | 4,0 mg |
| Milchzucker | (2) | 27,0 mg |
| Maisstärke | (3) | 14,5 mg |
| Polyvinylpyrrolidon | (4) | 4,0 mg |
| Magnesiumstearat | (5) | 0,5 mg |
| | | 50,0 mg |

Herstellung :

Die Stoffe 1-3 werden mit einer wäßrigen Lösung von 4 gleichmäßig befeuchtet, durch 1 mm-Maschenweite gesiebt, getrocknet und erneut durch 1 mm-Maschenweite gesiebt. Nach Zumischen von 5 wird die Mischung zu Dragéekernen verpreßt.

Dragéekerne : 5 mm 0, bikonvex, rund

Dragierung :

Übliche Zuckerdragierung auf 70 mg Endgewicht.

Beispiel II

Tabletten mit 8 mg 8-Methylthio-2-piperazino-4-pyrrolidino-pyrimido[5,4-d]pyrimidin

Tablette enthält :

| | |
|---|---|
| Wirksubstanz | 8,0 mg |
| Milchzucker | 23,0 mg |
| Maisstärke | 14,5 mg |
| Polyvinylpyrrolidon | 4,0 mg |
| Magnesiumstearat | 0,5 mg |
| | 50,0 mg |

Herstellung :
Analog den Dragéekernen.
Tablettenbeschreibung :

Gewicht : 50 mg
Durchmesser : 5 mm, biplan, beidseitige Facette

## Beispiel III

Suppositorien zu 25 mg 8-Methylthio-2-piperazino-4-pyrrolidino-pyrimido[4,5-d]pyrimidin

Zäpfchen enthält :

| | |
|---|---|
| Wirksubstanz | 0,025 g |
| Hartfett (z. B. Witepsol H 19 und Witepsol H 45) | 1,675 g |
| | 1,700 g |

Herstellung :

Das Hartfett wird geschmolzen. Bei 38 °C wird die gemahlene Wirksubstanz in der Schmelze homogen dispergiert. Es wird auf 35 °C abgekühlt und in schwach vorgekühlte Suppositorienformen ausgegossen.
Zäpfchengewicht : 1,7 g

## Beispiel IV

Suspension mit 8 mg 8-Methylthio-2-piperazino-4-pyrrolidino-pyrimido[5,4-d]pyrimidin

100 ml Suspension enthalten :

| | | |
|---|---|---|
| Wirksubstanz | | 0,16 g |
| Carboxymethylcellulose | | 0,1 g |
| p-Hydroxybenzoesäuremethylester | | 0,05 g |
| p-Hydroxybenzoesäurepropylester | | 0,01 g |
| Rohrzucker | | 10,0 g |
| Glycerin | | 5,0 g |
| Sorbitlösung 70 % | | 20,0 g |
| Aroma | | 0,3 g |
| Wasser dest. | ad | 100,0 ml |

Herstellungsverfahren :

Dest. Wasser wird auf 70 °C erhitzt. Hierin wird unter Rühren p-Hydroxybenzoesäuremethylester und -propylester sowie Glycerin und Carboxymethylcellulose gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.

## Beispiel V

Tabletten mit 100 mg 8-Methylthio-2-piperazino-4-pyrrolidino-pyrimido[5,4-d]pyrimidin

Zusammensetzung :
1 Tablette enthält :

| | |
|---|---|
| Wirkstoff | 100,0 mg |
| Milchzucker | 80,0 mg |
| Maisstärke | 34,0 mg |
| Polyvinylpyrrolidon | 4,0 mg |
| Magnesiumstearat | 2,0 mg |
| | 220,0 mg |

Herstellungsverfahren :

Wirkstoff, Milchzucker und Stärke werden gemischt und mit einer wäßrigen Lösung des Polyvinylpyrrolidons gleichmäßig befeuchtet. Nach Siebung der feuchten Masse (2,0 mm-Maschenweite) und Trocknen im Hordentrockenschrank bei 50 °C wird erneut gesiebt (1,5 mm-Maschenweite) und das Schmiermittel zugemischt. Die preßfertige Mischung wird zu Tabletten verarbeitet.
Tablettengewicht : 220 mg
Durchmesser : 10 mm, biplan mit beidseitiger Facette und einseitiger Teilkerbe.

Beispiel VI

Hartgelatine-Kapseln mit 150 mg 8-Methylthio-2-piperazino-4-pyrrolidino-pyrimido[5,4-d]pyrimidin

| | | |
|---|---|---|
| Kapsel enthält: | | |
| Wirkstoff | | 150,0 mg |
| Maisstärke getr. | ca. | 180,0 mg |
| Milchzucker pulv. | ca. | 87,0 mg |
| Magnesiumstearat | | 3,0 mg |
| | ca. | 420,0 mg |

Herstellung

Der Wirkstoff wird mit den Hilfsstoffen vermengt, durch ein Sieb von 0,75 mm-Maschenweite gegeben und in einem geeigneten Gerät homogen gemischt.
Die Endmischung wird in Hartgelatine-Kapseln der Größe 1 abgefüllt.
Kapselfüllung : ca. 320 mg
Kapselhülle : Hartgelatine-Kapsel Größe 1.

Beispiel VII

Suppositorien mit 150 mg 8-Methylthio-2-piperazino-4-pyrrolidino-pyrimido[5,4-d]pyrimidin

| | |
|---|---|
| 1 Zäpfchen enthält : | |
| Wirkstoff | 150,0 mg |
| Polyäthylenglykol 1500 | 550,0 mg |
| Polyäthylenglykol 6000 | 460,0 mg |
| Polyoxyäthylensorbitanmonostearat | 840,0 mg |
| | 2 000,0 mg |

Herstellung

Nach dem Aufschmelzen der Suppositorienmasse wird der Wirkstoff darin homogen verteilt und die Schmelze in vorgekühlte Formen gegossen.

Beispiel VIII

Suspension mit 50 mg 8-Methylthio-2-piperazino-4-pyrrolidino-pyrimido[5,4-d]pyrimidin

| | | |
|---|---|---|
| 100 ml Suspension enthalten : | | |
| Wirkstoff | | 1,0 g |
| Carboxymethylcellulose-Na-Salz | | 0,1 g |
| p-Hydroxybenzoesäuremethylester | | 0,05 g |
| p-Hydroxybenzoesäurepropylester | | 0,01 g |
| Rohrzucker | | 10,0 g |
| Glycerin | | 5,0 g |
| Sorbitlösung 70 %ig | | 20,0 g |
| Aroma | | 0,3 g |
| Wasser dest. | ad | 100 ml |

Herstellung

Dest. Wasser wird auf 70 °C erhitzt. Hierin wird unter Rühren p-Hydroxybenzoesäuremethylester und -propylester sowie Glycerin und Carboxymethylcellulose-Natriumsalz gelöst. Es wird auf Raumtemperatur abgekühlt und unter Rühren der Wirkstoff zugegeben und homogen dispergiert. Nach Zugabe und Lösen des Zuckers, der Sorbitlösung und des Aromas wird die Suspension zur Entlüftung unter Rühren evakuiert.
5 ml Suspension enthalten 50 mg Wirkstoff.

Beispiel IX

Tabletten mit 150 mg 8-Methylthio-2-piperazino-4-pyrrolidino-pyrimido[5,4-d]pyrimidin

Zusammensetzung :
1 Tablette enthält :

| | |
|---|---|
| Wirksubstanz | 150,0 mg |
| Milchzucker pulv. | 89,0 mg |
| Maisstärke | 40,0 mg |
| Kolloide Kieselgelsäure | 10,0 mg |
| Polyvinylpyrrolidon | 10,0 mg |
| Magnesiumstearat | 1,0 mg |
| | 300,0 mg |

Herstellung

Die mit Milchzucker, Maisstärke und Kieselsäure gemischte Wirksubstanz wird mit einer 20 %igen wäßrigen Polyvinylpyrrolidonlösung befeuchtet und durch ein Sieb mit 1,5 mm-Maschenweite geschlagen.

Das bei 45 °C getrocknete Granulat wird nochmals durch dasselbe Sieb gerieben und mit der angegebenen Menge Magnesiumstearat gemischt. Aus der Mischung werden Tabletten gepreßt.

Tablettengewicht : 300 mg
Stempel : 10 mm, flach

Beispiel X

Dragées mit 75 mg 8-Methylthio-2-piperazino-4-pyrrolidino-pyrimido[5,4-d]pyrimidin

1 Dragéekern enthält :

| | |
|---|---|
| Wirksubstanz | 75,0 mg |
| Calciumphosphat | 93,0 mg |
| Maisstärke | 35,5 mg |
| Polyvinylpyrrolidon | 10,0 mg |
| Hydroxypropylmethylcellulose | 15,0 mg |
| Magnesiumstearat | 1,5 mg |
| | 230,0 mg |

Herstellung

Die Wirksubstanz wird mit Calciumphosphat, Maisstärke, Polyvinylpyrrolidon, Hydroxypropylmethylcellulose und der Hälfte der angegebenen Menge Magnesiumstearat gemischt. Auf einer Tablettiermaschine werden Preßlinge mit einem Durchmesser von ca. 13 mm hergestellt, diese werden auf einer geeigneten Maschine durch ein Sieb 1,5 mm-Maschenweite gerieben und mit der restlichen Menge Magnesiumstearat vermischt. Dieses Granulat wird auf einer Tablettiermaschine zu Tabletten mit der gewünschten Form gepreßt.

Kerngewicht : 230 mg
Stempel : 9 mm, gewölbt

Die so hergestellten Dragéekerne werden mit einem Film überzogen, der im wesentlichen aus Hydroxypropylmethylcellulose besteht. Die fertigen Filmdragées werden mit Bienenwachs geglänzt.

Dragéegewicht : 245 mg.

Selbstverständlich können alle übrigen Verbindungen der allgemeinen Formel I als Wirkstoffe in den vorstehenden galenischen Zubereitungen eingesetzt werden.

**Patentansprüche**

1. 8-Alkylthio-2-piperazino-pyrimido[5,4-d]pyrimidine der allgemeinen Formel I

(Siehe Formel I Seite 13 f.)

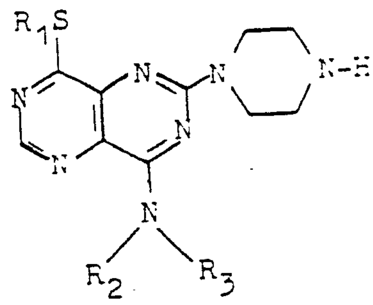

(I)

in der

$R_1$ eine Alkylgruppe mit 1 bis 3 Kohlenstoffatomen,

$R_2$ ein Wasserstoffatom, eine ab dem Kohlenstoff 2 gegebenenfalls durch eine Hydroxygruppe substituierte Alkylgruppe mit 1 bis 6 Kohlenstoffatomen, eine Cycloalkylgruppe mit 5 bis 7 Kohlenstoffatomen, eine Allyl-, Phenyl- oder Benzylgruppe,

$R_3$ eine Allylgruppe, eine ab dem Kohlenstoffatom 2 gegebenenfalls durch eine Hydroxygruppe substituierte Alkylgruppe mit 1 bis 6 Kohlenstoffatomen oder eine Cycloalkylgruppe mit 5 bis 7 Kohlenstoffatomen oder

$R_2$ und $R_3$ zusammen mit dem dazwischenliegenden Stickstoffatom eine geradkettige Alkyleniminogruppe mit 2 bis 8 Kohlenstoffatomen bedeuten, und deren Säureadditionssalze.

2. 8-Alkylthio-2-piperazino-pyrimido[5,4-d]pyrimidine der allgemeinen Formel I gemäß Anspruch 1, in der

$R_1$ wie im Anspruch 1 definiert ist,

$R_2$ ein Wasserstoffatom, eine Allylgruppe oder eine Alkylgruppe mit 1 bis 4 Kohlenstoffatomen,

$R_3$ eine Alkylgruppe mit 1 bis 6 Kohlenstoffatomen, eine Allyl-, Cyclohexyl-, Phenyl-, Benzyl-, 2-Hydroxyethyl-, 2-Hydroxy-n-propyl- oder 3-Hydroxy-n-propylgruppe oder

$R_2$ und $R_3$ zusammen mit dem dazwischenliegenden Stickstoffatom eine Pyrrolidino-, Piperidino-, Hexamethylenimino-, oder Heptamethyleniminogruppe bedeuten, und deren Säureadditionssalze.

3. 8-Alkylthio-2-piperazino-pyrimido[5,4-d]pyrimidine der allgemeinen Formel I gemäß Anspruch 1, in der

$R_1$ eine Methylgruppe,

$R_2$ ein Wasserstoffatom, eine Methyl- oder Ethylgruppe,

$R_3$ eine Alkylgruppe mit 1 bis 6 Kohlenstoffatomen oder

$R_2$ und $R_3$ zusammen mit dem dazwischenliegenden Stickstoffatom eine Pyrrolidino-, Piperidino- oder Hexamethyleniminogruppe bedeuten, und deren Säureadditionssalze.

4. 8-Alkylthio-2-piperazino-pyrimido[5,4-d]pyrimidine der allgemeinen Formel I gemäß Anspruch 1, in der

$R_1$ die Methylgruppe und

$R_2$ und $R_3$ zusammen mit dem dazwischenliegenden Stickstoffatom die Dimethylamino-, Diethylamino-, Pyrrolidino-, Piperidino- oder Hexamethyleniminogruppe bedeuten, und deren Säureadditionssalze.

5. 8-Methylthio-2-piperazino-4-pyrrolidino-pyrimido[5,4-d]pyrimidin und dessen Säureadditionssalze.

6. Physiologisch verträgliche Säureadditionssalze der Verbindungen gemäß den Ansprüchen 1 bis 5 mit physiologisch verträglichen anorganischen oder organischen Säuren.

7. Arzneimittel, enthaltend eine Verbindung gemäß den Ansprüchen 1 bis 5 oder ein physiologisch verträgliches Säureadditionssalz gemäß Anspruch 6 neben einem oder mehreren inerten Trägerstoffen und/oder Verdünnungsmitteln.

8. Arzneimittel gemäß Anspruch 7 zur Prophylaxe thrombo-embolischer Erkrankungen, zur Prophylaxe der Arteriosklerose, zur Metastasenprophylaxe und zur Hemmung des Tumorwachstums.

9. Verfahren zur Herstellung eines Arzneimittels gemäß den Ansprüchen 7 und 8, dadurch gekennzeichnet, daß eine Verbindung gemäß den Ansprüchen 1 bis 5 oder ein physiologisch verträgliches Säureadditionssalz gemäß Anspruch 6 in einen oder mehreren inerten Trägerstoffen und/oder Verdünnungsmitteln eingearbeitet wird.

10. Verfahren zur Herstellung der Verbindungen gemäß den Ansprüchen 1 bis 6, dadurch gekennzeichnet, daß ein Pyrimido[5,4-d]pyrimidin der allgemeinen Formel II

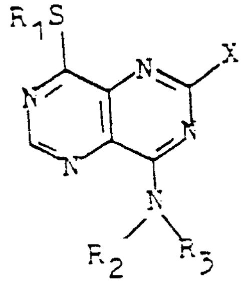

(II)

in der

$R_1$ bis $R_3$ wie in den Ansprüchen 1 bis 5 definiert sind und

X eine nukleofuge Austrittsgruppe darstellt, mit einem Piperazin der allgemeinen Formel III

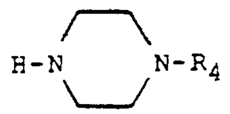

(III)

in der

$R_4$ ein Wasserstoffatom oder eine leicht abspaltbare Schutzgruppe darstellt, umgesetzt und erforderlichenfalls anschließend ein verwendeter Schutzrest abgespalten wird und eine so erhaltene Verbindung der allgemeinen Formel I gewünschtenfalls anschließend in ihr Säureadditionssalz, insbesondere in ihr physiologisch verträgliches Säureadditionssalz, mit einer anorganischen oder organischen Säure übergeführt wird.

## Claims

1. 8-Alkylthio-2-piperazino-pyrimido[5,4-d]pyrimidines of general formula I

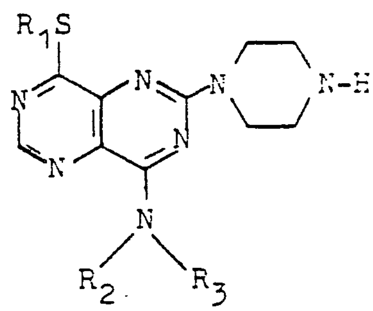

(I)

wherein

$R_1$ represents a $C_{1-3}$ alkyl group,

$R_2$ represents a hydrogen atom, a $C_{1-6}$ alkyl group optionally substituted by a hydroxy group from the second or higher carbon atom, a $C_{5-7}$ cycloalkyl group or an allyl, phenyl or benzyl group,

$R_3$ represents an allyl group, a $C_{1-6}$ alkyl group optionally substituted by a hydroxy group from the second or higher carbon atom, or a $C_{5-7}$ cycloalkyl group or

$R_2$ and $R_3$ together with the nitrogen atom between them represent a straight-chained alkyleneimino group with 2 to 8 carbon atoms, and the acid addition salts thereof.

2. 8-Alkylthio-2-piperazino-pyrimido[5,4-d]pyrimidines of general formula I according to claim 1, wherein

$R_1$ is defined as in claim 1,

$R_2$ represents a hydrogen atom, an allyl group or a $C_{1-4}$ alkyl group,

$R_3$ is a $C_{1-6}$ alkyl group, an allyl, cyclohexyl, phenyl, benzyl, 2-hydroxyethyl, 2-hydroxy-n-propyl or 3-hydroxy-n-propyl group or

$R_2$ and $R_3$ together with the nitrogen atom between them represent a pyrrolidino, piperidino, hexamethyleneimino or heptamethyleneimino group, and the acid addition salts thereof.

3. 8-Alkylthio-2-piperazino-pyrimido[5,4-d]pyrimidines of general formula I according to claim 1, wherein

$R_1$ represents a methyl group,

$R_2$ represents a hydrogen atom or a methyl or ethyl group,

$R_3$ represents a $C_{1-6}$ alkyl group or

$R_2$ and $R_3$ together with the nitrogen atom between them represent a pyrrolidino, piperidino or hexamethyleneimino group, and the acid addition salts thereof.

4. 8-Alkylthio-2-piperazino-pyrimido[5,4-d]pyrimidines of general formula I according to claim 1, wherein

$R_1$ represents a methyl group and

$R_2$ and $R_3$ together with the nitrogen atom between them represent a dimethylamino, diethylamino, pyrrolidino, piperidino or hexamethyleneimino group, and the acid addition salts thereof.

5. 8-Methylthio-2-piperazino-4-pyrrolidinopyrimido[5,4-d]pyrimidine and the acid addition salts thereof.

6. Physiologically acceptable acid addition salts of the compounds according to claims 1 to 5 with physiologically acceptable organic or inorganic acids.

7. Pharmaceutical compositions containing a compound as claimed in claims 1 to 5 or a physiologically acceptable acid addition salt as claimed in claim 6 together with one or more inert carriers and/or diluents.

8. Pharmaceutical compositions according to claim 7 for the prevention of thromboembolic diseases, arteriosclerosis and metastasis and for inhibiting tumour growth.

9. Process for preparing a pharmaceutical composition as claimed in claims 7 and 8, characterised in that a compound as claimed in claims 1 to 5 or a physiologically acceptable acid addition salt as claimed in claim 6 is incorporated in one or more inert carriers and/or diluents.

10. Process for preparing the compounds as claimed in claims 1 to 6, characterised in that a pyrimido[5,4-d]pyrimidine of general formula II

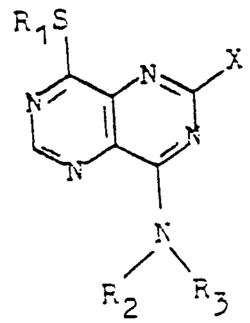

(II)

wherein

$R_1$ to $R_3$ are defined as in claims 1 to 5 and

X represents a nucleophobic leaving group, is reacted with a piperazine of general formula III

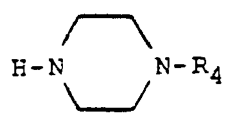

(III)

wherein

$R_4$ represents a hydrogen atom or an easily cleavable protecting group and if necessary any protecting group used is subsequently split off and a compound of general formula I thus obtained is converted, if desired, into an acid addition salt thereof, more particularly a physiologically acceptable acid addition salt thereof, with an inorganic or organic acid.

## Revendications

1. 8-alkylthio-2-pipérazino-pyrimido[5,4-d]pyrimidines de formule générale I

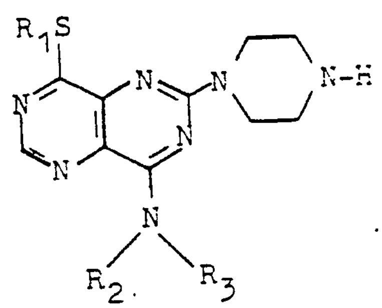

(I)

dans laquelle

$R_1$ représente un groupe alkyle avec 1 à 3 atomes de carbone,

$R_2$ représente un atome d'hydrogène, un groupe alkyle avec 1 à 6 atomes de carbone, éventuellement substitué, à partir de l'atome de carbone 2, par un groupe hydroxy, ou représente un groupe cycloalkyle avec 5 à 7 atomes de carbone, un groupe allyle, phényle ou benzyle,

$R_3$ représente un groupe allyle, un groupe alkyle avec 1 à 6 atomes de carbone, éventuellement substitué, à partir de l'atome de carbone 2, par un groupe hydroxy, ou représente un groupe cycloalkyle avec 5 à 7 atomes de carbone, ou

$R_2$ et $R_3$, ensemble avec l'atome d'azote intermédiaire, représentent un groupe alkylènimino rectiligne avec 2 à 8 atomes de carbone, et leurs sels d'addition d'acides.

2. 8-alkylthio-2-pipérazino-pyrimido[5,4-d]pyrimidines de formule générale I selon la revendication 1, dans laquelle

$R_1$ est défini comme à la revendication 1,

$R_2$ représente un atome d'hydrogène, un groupe allyle ou un groupe alkyle avec 1 à 4 atomes de carbone,

$R_3$ représente un groupe alkyle avec 1 à 6 atomes de carbone, un groupe allyle, cyclohexyle, phényle, benzyle, 2-hydroxyéthyle, 2-hydroxy-n-propyle ou 3-hydroxy-n-propyle ou

$R_2$ et $R_3$, ensemble avec l'atome d'azote intermédiaire, représentent un groupe pyrrolidino, pipéridino, hexaméthylènimino, ou heptaméthylènimino, et leurs sels d'addition d'acides.

3. 8-alkylthio-2-pipérazino-pyrimido[5,4-d]pyrimidines de formule générale I selon la revendication 1, dans laquelle

$R_1$ représente un groupe méthyle,

$R_2$ représente un atome d'hydrogène, un groupe méthyle ou éthyle,

$R_3$ représente un groupe alkyle avec 1 à 6 atomes de carbone, ou

$R_2$ et $R_3$, ensemble avec l'atome d'azote intermédiaire, représentent un groupe pyrrolidino, pipéridino ou hexaméthylènimino, et leurs sels d'addition d'acides.

4. 8-alkylthio-2-pipérazino-pyrimido[5,4-d]pyrimidines de formule générale I selon la revendication 1, dans laquelle

$R_1$ représente le groupe méthyle,

$R_2$ et $R_3$, ensemble avec l'atome d'azote intermédiaire, représentent le groupe diméthylamino, diéthylamino, pyrrolidino, pipéridino ou hexaméthylènimino, et leurs sels d'addition d'acides.

5. La 8-méthylthio-2-pipérazino-4-pyrolidino-pyrimido[5,4-d]pyrimidine et ses sels d'addition d'acides.

6. Sels d'addition d'acides physiologiquement supportables des composés selon les revendications 1 à 5 avec des acides minéraux ou organiques physiologiquement supportables.

7. Médicament contenant un composé selon les revendications 1 à 5 ou un sel d'addition d'acide physiologiquement supportable selon la revendication 6, conjointement à un ou plusieurs excipients et/ou diluants inertes.

8. Médicament selon la revendication 7 pour la prophylaxie des maladies thrombo-emboliques, pour la prophylaxie de l'artériosclérose, pour la prophylaxie des métastases et pour l'inhibition de la croissance des tumeurs.

9. Procédé pour la fabrication d'un médicament selon les revendications 7 et 8, caractérisé en ce qu'on introduit un composé selon les revendications 1 à 5 ou un sel d'addition d'acide physiologiquement supportable selon la revendication 6 dans un ou plusieurs excipients et/ou diluants inertes.

10. Procédé pour la préparation des composés selon les revendications 1 à 6, caractérisé en ce qu'on fait réagir une pyrimido[5,4-d]pyrimidine de formule générale II

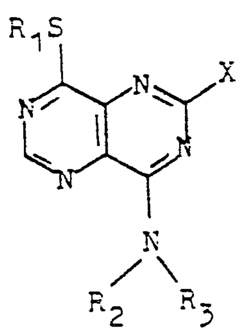

(II)

dans laquelle

$R_1$ à $R_3$ sont définis comme dans les revendications 1 à 5, et

X représente un groupe partant nucléofuge, avec une pipérazine de formule générale III

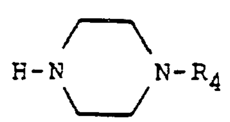

(III)

dans laquelle

$R_4$ représente un atome d'hydrogène ou un groupe protecteur facilement clivable et, si nécessaire, on clive ensuite un radical protecteur utilisé et on transforme un composé ainsi obtenu de formule générale I, si on le désire, ensuite en son sel d'addition d'acide, en particulier en son sel d'addition d'acide physiologiquement supportable, avec un acide minéral ou organique.